# EUROPEAN PATENT APPLICATION

(11) **EP 4 446 438 A1**
(43) Date of publication of application: **16.10.2024**
(21) Application number: 22904695.8
(22) Date of filing: 08.12.2022
(51) Int. Cl.: C12Q 1/6876, C12Q 1/70

(54) **TAGGED LOOP PRIMER COMPRISING SINGLE-STRANDED INTERACTIVE DOUBLE LABELING SYSTEM, AND ISOTHERMAL NUCLEIC ACID AMPLIFICATION METHOD USING SAME**

(30) Priority: 10.12.2021 KR 20210177086
(71) Applicant: Seegene, Inc., Seoul 05548 (KR)
(72) Inventor: KO, Ohsung, Hanam-si Gyeonggi-do 12913 (KR); PARK, Hyun Shin, Seoul 05658 (KR); HAN, Seong Hyun, Seoul 06146 (KR); PARK, Jihoon, Hanam-si Gyeonggi-do 12942 (KR); LEE, Hyobeen, Seoul 05058 (KR)
(74) Representative: Kilger, Ute
(86) International application number: PCT/KR2022/019959
(87) International publication number: WO 2023/106866

(57) **Abstract**

The present disclosure relates to a tagged loop primer comprising a single-stranded interactive dual labeling system and an isothermal nucleic acid amplification method using the same. The tagged loop primer for a loop-mediated isothermal amplification reaction of the present disclosure can be easily prepared, and furthermore, Using the tagged loop primer of the present disclosure enables the effective simultaneous detection of both single target nucleic acids and multiple target nucleic acids.

## Description

### Technical Field

The present disclosure relates to a tagged loop primer comprising a single-stranded interactive dual labeling system and a nucleic acid isothermal amplification method using the same.

### Background Art

Nucleic acid amplification technology is a technology mainly used in the fields of molecular biology and biotechnology which detects and analyzes a small amount of target nucleic acid present in a sample. PCR (Polymerase chain reaction) technology is most commonly used for nucleic acid amplification, which repeats a series of processes of denaturing a double-stranded DNA into a single-stranded DNA, annealing a primer to the single-stranded DNA, and extending the annealed primer by a polymerase.

The real-time PCR method using a fluorescent material is a method of detecting an increase in fluorescence intensity due to nucleic acid amplification during the PCR process. The real-time PCR method has an advantage of being able to perform multiplex detection by using different fluorescent dyes for each target, but has a disadvantage of requiring expensive equipment and taking a long time to detect.

As an alternative to the PCR method, an isothermal amplification method has been developed that does not require expensive real-time PCR equipment and can detect target nucleic acids within a shorter time than PCR. Examples of isothermal amplification methods include rolling circle amplification (RCA, M. M. Ali, F. Li, Z. Zhang, K. Zhang, D.-K. Kang, J. A. Ankrum, X. C. Le and W. Zhao, Chem. Soc. Rev., 2014, 43, 3324-3341.), loop-mediated isothermal amplification (LAMP, Y. Mori, H. Kanda and T. Notomi, J. Infect. Chemother., 2013, 19, 404-411), recombinase polymerase amplification (RPA, J. Li, J. Macdonald and F. von Stetten, Analyst, 2018, 144, 31-67), nucleic acid sequence based amplification (NASBA, A. Borst, J. Verhoef, E. Boel and A. C. Fluit, Clin. Lab., 2002, 48, 487-492), strand displacement amplification (SDA, B. J. Toley, I. Covelli, Y. Belousov, S. Ramachandran, E. Kline, N. Scarr, N. Vermeulen, W. Mahoney, B. R. Lutz and P. Yager, Analyst, 2015, 140, 7540-7549), helicase dependent amplification (HAD, M. Vincent, Y. Xu and H. Kong, EMBO Rep., 2004, 5, 795-800), and transcription-mediated amplification (TMA, L. Comanor, Am. J. Gastroenterol., 2001, 96, 2968-2972).

Among the isothermal amplification methods, LAMP method is widely used. LAMP was first developed by Notomi et al. (T. Notomi, H. Okayama, H. Masubuchi, T. Yonekawa, K. Watanabe, N. Amino and T. Hase, Nucleic Acids Res., 2000, 28, E63) in 2000, and was optimized for accelerated amplification by Nagamine et al. (K. Nagamine, T. Hase and T. Notomi, Mol. Cell. Probes, 2002, 16, 223-229) through the use of an additional primer.

The standard detection method for LAMP is turbidity measurement by precipitation of magnesium pyrophosphate (Y. Mori, K. Nagamine, N. Tomita and T. Notomi, Biochem. Biophys. Res. Commun., 2001, 289, 150-154), and other methods include gel electrophoresis, metal indicators for calcium, colorimetric LAMP, Coffee-Ring effect on colloid-crystal substrates, paper-based rapid detection of LAMP magnetic bead aggregates, melting and annealing curve analysis, intercalating fluorescent dyes such as SYBR green, bioluminescence or electrochemical luminescence via pyrophosphate conversion, and the like. Recently, a method for detecting fluorescence specific to a target nucleic acid amplification sequence using an assimilating probe has been developed (WO2011-163425).

In general, isothermal amplification methods such as the LAMP method can amplify genes at one temperature within a short period of time (usually within 1 hour) and do not require expensive equipment compared to real-time PCR, and thus has been applied to point of care testing.

Regarding the LAMP method, there has been a persistent demand for easily manufacturable and optimized primer designs that can enhance the detection efficiency of isothermal amplification reactions.

Throughout this application, various patents and publications are referenced and citations are provided in parentheses. The disclosure of these patents and publications in their entities are hereby incorporated by references into this application in order to more fully describe this invention and the state of the art to which this invention pertains.

### Disclosure

### Technical Problem

The present inventors endeavored to develop a single-stranded loop primer that can be used in a LAMP (Loop-mediated isothermal amplification) reaction method and to which a new signaling scheme that has not been applied to the existing LAMP method is applied. As a result, the inventors have completed the present invention by demonstrating that the effective detection of one or more target nucleic acids is achievable when performing LAMP reaction using a tagged loop primer, which comprises a 3' -targeting portion that forms a complementary bond to the target nucleic acid and a 5' -tagging portion that does not form a complementary bond to the target nucleic acid, and in which a reporter molecule and a quencher molecule are linked to the 5' -tagging portion, spaced apart from each other at a predetermined interval.

Therefore, it is an object of the present invention to provide a tagged loop primer for a loop-mediated isothermal amplification reaction.

It is another object of the present invention to provide a method for detecting a target nucleic acid using the tagged loop primer.

It is another object of the present invention to provide a composition for a loop-mediated isothermal amplification reaction comprising the tagged loop primer.

### Solution to Problem

According to one aspect of the present disclosure, provided is a tagged loop primer for detecting a target nucleic acid having, in a 3' to 5' direction, (i) F3c, F2c, F1c, B1, B2 and B3 or (ii) B3c, B2c, B1c, F1, F2 and F3, the tagged loop primer comprising:
(i) a 3' -targeting portion comprising a nucleotide sequence complementary to all or part of a sequence between the B1 and the B2 or between the F1 and the F2 of the target nucleic acid; and
(ii) a 5' -tagging portion comprising a nucleotide sequence non-complementary to the target nucleic acid:
   wherein the 5' -tagging portion comprises a reporter molecule and a quencher molecule,
   wherein in the absence of the target nucleic acid, the reporter molecule and the quencher molecule in the 5' -tagging portion are in close proximity to each other to allow the quencher molecule to quench a signal from the reporter molecule,
   wherein in the presence of the target nucleic acid, the reporter molecule and the quencher molecule in the 5' -tagging portion are separated from each other to allow the quencher molecule to unquench the signal from the reporter molecule.

According to an embodiment of the present disclosure, in the absence of the target nucleic acid, the 5-tagging portion is spatially folded.

According to an embodiment of the present disclosure, in the presence of the target nucleic acid, the 5-tagging portion is spatially unfolded.

According to an embodiment of the present disclosure, in the absence of the target nucleic acid, the 5-tagging portion forms a hairpin structure.

According to an embodiment of the present disclosure, in the presence of the target nucleic acid, the hairpin structure of the 5-tagging portion is disrupted.

According to an embodiment of the present disclosure, the tagged loop primer is 20 to 150 nucleotides in length.

According to an embodiment of the present disclosure, the 3' - targeting portion has a nucleotide length sufficient to hybridize with the target nucleic acid in a LAMP reaction.

According to an embodiment of the present disclosure, the 3' - targeting portion is 10 to 100 nucleotides in length.

According to an embodiment of the present disclosure, the 3' - targeting portion has a Tm value of 40°C to 70°C.

According to an embodiment of the present disclosure, the 5' -tagging portion has a nucleotide length such that the reporter molecule and the quencher molecule therein are apart from each other to allow the quencher molecule to unquench the signal from the reporter molecule.

According to an embodiment of the present disclosure, the 5' -tagging region is at least 10 nucleotides in length.

According to an embodiment of the present disclosure, the reporter molecule and the quencher molecule are located at least 10 nucleotides apart from each other.

According to an embodiment of the present disclosure, one of the reporter molecule and the quencher molecule is linked to a 5' -end of the 5' -tagging portion, and the other is linked to an internal deoxythymidine (dT) present in the 5' -tagging portion.

According to one aspect of the present disclosure, provided is a method for detecting one or more target nucleic acids, comprising performing a loop-mediated isothermal amplification (LAMP) reaction using one or more tagged loop primers.

According to an embodiment of the present disclosure, the LAMP reaction is performed at a temperature between 50°C and 75°C.

According to an embodiment of the present disclosure, the target nucleic acid is from a virus.

According to an embodiment of the present disclosure, the virus is an RNA virus.

According to an embodiment of the present disclosure, the LAMP reaction further uses a conventional, untagged loop primer.

According to one aspect of the present disclosure, provided is a composition for a loop-mediated isothermal amplification reaction, comprising a tagged loop primer.

### Advantageous Effects

The features and advantages of the present invention will be summarized as follows:
(a) The tagged loop primer of the present disclosure is characterized by comprising both a reporter molecule and a quencher molecule in its single-strand, and is able to simultaneously serve as a probe as well as a primer.
(b) The tagged loop primer according to the present disclosure does not require a process of synthesizing a pair of probes as in a conventional method of using two probes, for example, an assimilating probe method, does not need to be designed to maintain a hybridized state of the two probes at an isothermal amplification reaction temperature, and has an advantage of blocking a possibility of noise generation due to non-specific disruption of partial complementary bonds between the two probes.
(c) Therefore, the tagged loop primer according to the present disclosure has the advantages of being easily prepared, and effectively detecting a plurality of target nucleic acids simultaneously as well as a single target nucleic acid.

### Description of Drawings

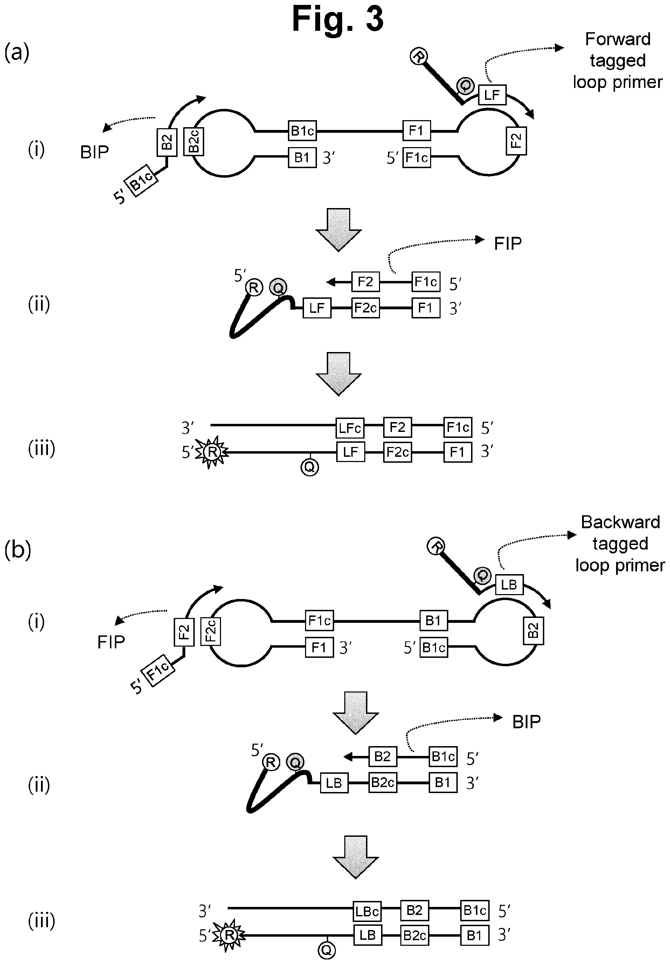

Fig. 1 schematically shows an embodiment of a loop-mediated isothermal amplification (LAMP) method using four types of primers (F3, B3, FIP, and BIP) and two types of conventional untagged loop primers (LF and LB).
Fig. 2 schematically shows a structure of the tagged loop primer according to the present disclosure.
Fig. 2 (a) shows (i) a 5' -tagging portion comprising a reporter molecule and a quencher molecule and (ii) a 3' -targeting portion comprising a nucleotide sequence complementary to all or part of a sequence between B1 and B2 or between F1 and F2 of a target nucleic acid in a tagged loop primer.
Fig. 2 (b) shows a quenching effect occurring when the 5' -tagging portion of the tagged loop primer forms a spatially folded structure like a random coil, thereby bring the reporter molecule and the quencher molecule into close proximity to each other.
Fig. 2 (c) shows a quenching effect occurring when the 5' -tagging portion of the tagged loop primer forms a hairpin structure, thereby bring the reporter molecule and the quencher molecule into close proximity to each other.
Fig. 3 schematically shows an embodiment of a loop-mediated isothermal amplification (LAMP) method using the tagged loop primer according to the present disclosure.
Fig. 3 (a) is an embodiment of a LAMP method using a forward tagged loop primer (tagged LF) which comprises the 3' -targeting portion comprising a nucleotide sequence complementary to all or part of a sequence between the F1 and the F2 of the target nucleic acid.
Fig. 3 (b) is an embodiment of a LAMP method using a reverse tagged loop primer (tagged LB) which comprises the 3' -targeting portion comprising a nucleotide sequence complementary to all or part of a sequence between the B1 and the B2 of the target nucleic acid.
As shown in (a) and (b) of Fig. 3, when the 5' -tagging portion of the tagged loop primer is single-stranded, it exists in the form of a random coil (or hairpin structure), so that the reporter molecule and the quencher molecule are in close proximity to each other to allow the quencher molecule to quench a signal from the reporter molecule (see (i) or (ii) of Fig. 3 (a) and (i) or (ii) of Fig. 3 (b)), whereas when the 5' -tagging portion is double-stranded through an LAMP reaction, the reporter molecule and the quencher molecule are separated from each other to allow the quencher molecule to unquench the signal from the reporter molecule (see (iii) of Fig. 3 (a) and (iii) of Fig. 3 (b)).

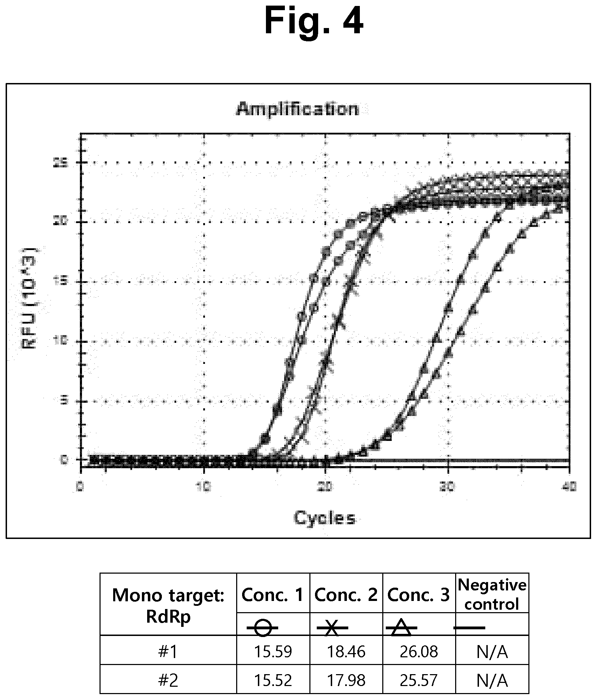

Fig. 4 shows the Mono RT-LAMP result for the RdRp gene as a target nucleic acid in Example 3.

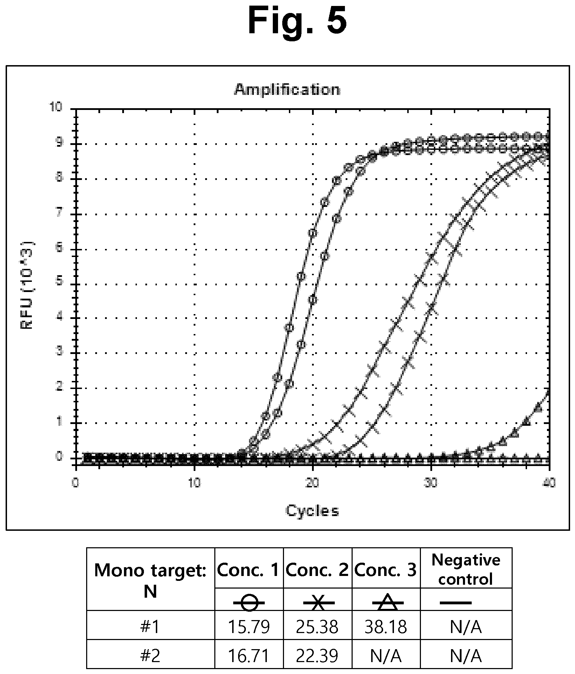

Fig. 5 shows the Mono RT-LAMP result for the N-gene as a target nucleic acid in Example 3.

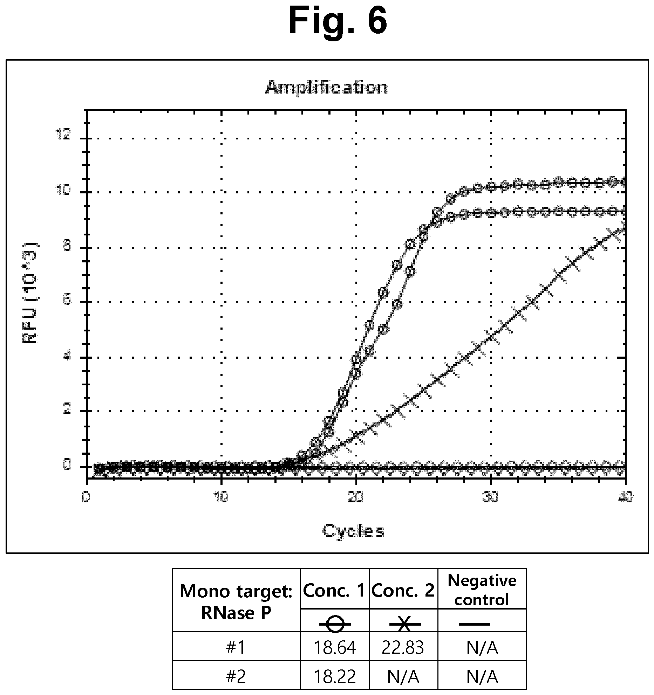

Fig. 6 shows the Mono RT-LAMP result for the RNase P gene as a target nucleic acid in Example 3.

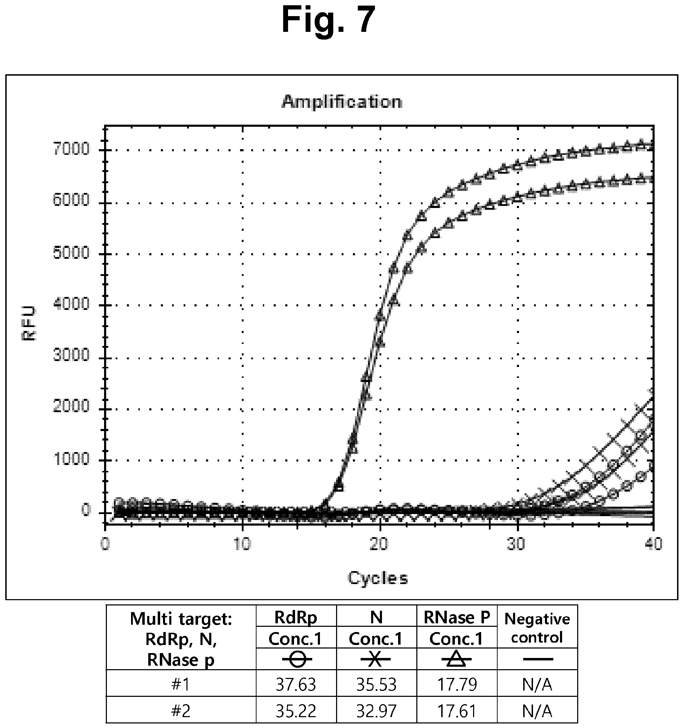

Fig. 7 shows the Multi RT-LAMP result for the RdRP gene, N gene and RNase P gene as target nucleic acids in Example 3.

### Best Mode

The present inventors endeavored to develop a single-stranded loop primer that can be used in a LAMP (Loop-mediated isothermal amplification) reaction method and to which a new signaling mechanism that has not been applied to the existing LAMP method is applied. As a result, the present inventors have found that the effective detection of one or more target nucleic acids is achievable when performing LAMP reaction using a tagged loop primer, which comprises a 3' -targeting portion that forms a complementary bond to the target nucleic acid and a 5' -tagging portion that does not form a complementary bond to the target nucleic acid, and in which a reporter molecule and a quencher molecule are linked to the 5' -tagging portion, spaced apart from each other at a predetermined interval.

According to an aspect of the present disclosure, there is provided a tagged loop primer for detecting a target nucleic acid having, in a 3' to 5' direction, (i) F3c, F2c, F1c, B1, B2 and B3 or (ii) B3c, B2c, B1c, F1, F2 and F3, the tagged loop primer comprising:
(i) a 3' -targeting portion comprising a nucleotide sequence complementary to all or part of a sequence between the B1 and the B2 or between the F1 and the F2 of the target nucleic acid; and
(ii) a 5' -tagging portion comprising a nucleotide sequence non-complementary to the target nucleic acid:
   wherein the 5' -tagging portion comprises a reporter molecule and a quencher molecule,
   wherein in the absence of the target nucleic acid, the reporter molecule and the quencher molecule in the 5' -tagging portion are in close proximity to each other to allow the quencher molecule to quench a signal from the reporter molecule,
   wherein in the presence of the target nucleic acid, the reporter molecule and the quencher molecule in the 5' -tagging portion are separated from each other to allow the quencher molecule to unquench the signal from the reporter molecule.

In the present disclosure, Loop-mediated isothermal amplification (LAMP) reaction is disclosed in detail in "LAMP, Y. Mori, H. Kanda and T. Notomi, J. Infect. Chemother., 2013, 19, 404-411" .

In the present disclosure, the loop-mediated isothermal amplification reaction is used as a meaning including a reverse transcription loop-mediated isothermal amplification (RT-LAMP).

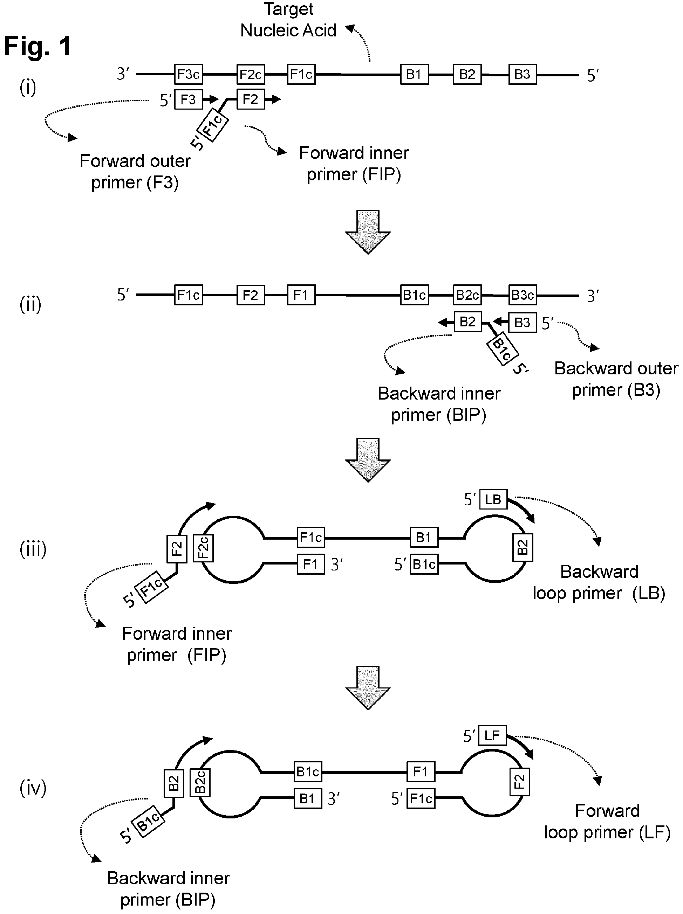

Loop-mediated isothermal amplification is a nucleic acid amplification method capable of amplifying a target nucleic acid with high sensitivity and specificity under isothermal conditions (Notomi, T. et al. 2000. Loop-Mediated Isothermal Amplification of DNA. Nucleic Acids Res 28, E63). The LAMP method uses a set of four to six primers specifically designed at different sites of the target nucleic acid and DNA polymerase having strand displacement activity (see Fig. 1).

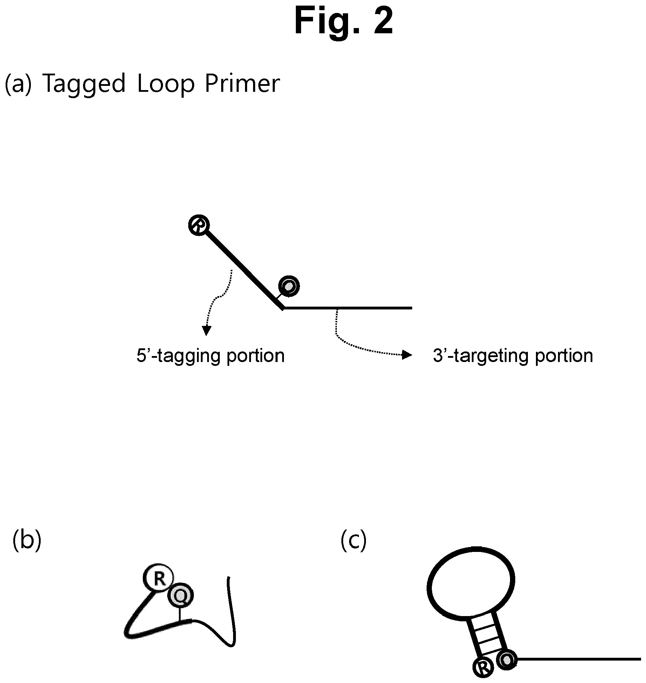

The tagged loop primer of the present disclosure is a primer used for a loop-mediated isothermal amplification reaction for detecting a target nucleic acid from a biological sample or a non-biological sample separated from a subject, and the presence or absence of the target nucleic acid in the sample can be rapidly determined through the loop-mediated isothermal amplification reaction using the tagged loop primer. The tagged loop primer of the present disclosure is characterized by being a single-stranded primer comprising both a reporter molecule and a quencher molecule, and is also described as "a single-stranded dual labels loop primer" . As described with reference to Fig. 2 (a), the tagged loop primer according to the present disclosure comprises (i) a 5' -tagging portion comprising a reporter molecule and a quencher molecule and (ii) a 3' -targeting portion comprising a nucleotide sequence complementary to all or part of a sequence between B1 and B2 or between F1 and F2 of the target nucleic acid.

As used herein, the term "between" as used in connection with two numerical values or locations, is to be interpreted as not including two numerical values or locations, while the term "to" is to be understood as including two numerical values or locations. For example, between B1 and B2 herein is interpreted as not including B1 and B2, and 10 to 100 is interpreted as including 10 and 100.

As used herein, the term "subject" refers to an individual suspected of comprising a target nucleic acid (e.g., a specific pathogen) to be detected using the method of the present disclosure. Examples of the subject include, but are not limited to, mammals such as a dog, a cat, a rodent, a primate, a human, and the like, and in particular, a human.

As used herein, the term "sample" may mean any analyte comprising or suspected to comprise a nucleic acid to be detected. For example, the sample includes a biological sample (e.g., cells, tissues, and body fluids) and a non-biological sample (e.g., food, water, and soil), and the biological sample may be, for example, viruses, bacteria, tissues, cells, blood (including whole blood, plasma, and serum), lymph, bone marrow fluid, saliva, sputum, swab, aspiration, milk, urine, feces, eye fluid, semen, brain extract, spinal fluid, joint fluid, thymus fluid, bronchial lavage fluid, ascites, or amniotic fluid, but is not limited thereto.

The sample may be used interchangeably with "specimen" herein.

According to an embodiment, the sample may be obtained from a subject, particularly a mammal, more particularly a human, and may be, for example, a swab, saliva, sputum, aspiration, bronchoalveolar lavage (BAL), gargle, or blood, but is not limited thereto.

In certain embodiments, the sample derived from the subject is a swab, saliva, or a mixture thereof.

In an embodiment, the swab is nasopharyngeal swab, nostril swab, oropharyngeal swab, oral swab, saliva swab, genital swab, rectal swab or a mixture of two or more thereof.

In one embodiment, the sample may be subjected to a nucleic acid extraction process known in the art for efficient amplification (i.e., LAMP reaction) (see Sambrook, J. et al., Molecular Cloning. A Laboratory Manual, 3rd ed. Cold Spring Harbor Press (2001)). The nucleic acid extraction process may vary depending on the type of sample. If necessary, a nucleic acid purification process may be added.

According to another embodiment, the sample may be directly analyzed without extracting the nucleic acid in the sample. For example, the methods disclosed in Pannacio et al. (Nucleic Acids Res. 1993 September 25; 21(19): 4656), Pandori et al. (BMC Infect Dis. 2006 Jun 24; 6: 104) and WO 2021-261864 may be used.

As used herein, the term "primer" refers to a single-stranded oligonucleotide used in an amplification reaction, which is a nucleic acid molecule that is extended by adding nucleotides to its 3'-end by covalent bonding in a nucleic acid amplification or synthesis reaction using polymerase.

Four types of primers (F3, B3, FIP, and BIP) are essentially required for the LAMP reaction, and two types of loop primers (LF and LB) may be additionally used to improve the reaction rate. The four primers consist of two outer primers and two inner primers, the two outer primers consist of a forward outer (F3) and a backward outer (B3) primer, and play a role in unwinding double-stranded DNA during the non-cyclic step. The two inner primers consist of a forward inner primer (FIP) and a backward inner primer (BIP), and each primer consists of a nucleotide sequence corresponding to a forward or a reverse sequence, respectively, enabling the generation of a loop essential for the LAMP reaction. The additional two loop primers consist of a forward loop (LoopF) primer and a backward loop (LoopB) primer, and hybridize with a sequence to which the inner primer does not hybridize, thereby accelerating the LAMP reaction.

The primer set used for LAMP is designed for six distinct regions (F3, F2, F1, B1c, B2c and B3) on the target nucleic acid, where F3 and B3 determine the size of the product amplified with two outer primers, FIP and BIP are two inner primers, FIP is a hybrid primer consisted of F1c and F2 sequences, and BIP is a hybrid primer consisted of B1c and B2 sequences.

The LAMP reaction largely includes a starting structure production step and a cycling amplification step, and does not include a step of denaturing double strand into single strand unlike PCR. In the starting structure production step, F2 of FIP binds to the F2c region to initiate extension. The BIP also proceeds in the same manner. F3 primer binds to the F3c region and initiates DNA synthesis in a strand displacement manner, and the DNA strand extending from FIP is replaced and released. The released single-strand forms a loop at the 5' -end portion through F1/F1c binding, and the BIP and B3 primers also undergo a synthesis reaction in the same manner, thereby forming a dumbbell-shaped single-stranded nucleic acid molecule with loops formed at both ends. Subsequently, DNA synthesis starts from the 3' end of the F1 region, and the cycling step proceeds. In the cycling amplification step, only two internal primers (optionally, along with a loop primer) are used. Initially, one internal primer binds to the loop, and DNA synthesis occurs via strand displacement, creating a new loop region. DNA synthesis then proceeds in a strand displacement manner using a newly generated single-stranded nucleic acid molecule having a plurality of loop regions as a template, thereby amplifying the target nucleic acid.

Briefly describing the LAMP reaction with reference to Fig. 1, in step (i) of Fig. 1, the forward inner primer (FIP) is hybridized with the target nucleic acid and extended, and the forward outer primer (F3) is hybridize with the outside thereof and extended, thereby causing strand displacement, and the extended strand of the FIP is separated into a single strand. In this case, the 5' -end portion of the extended strand of the single-stranded FIP forms a loop by hybridization between F1c and F1. In step (ii) of Fig. 1, the backward inner primer (BIP) is hybridize with the extended strand of the FIP generated in step (i) and extended, and the backward outer primer (B3) is hybridized with the outside thereof and extended, resulting in strand displacement, and the extended strand of the BIP is separated into a single strand. In this case, the extended strand of the single-stranded BIP forms a dumbbell shape in which its 5' -end region forms a loop by hybridization between F1 and F1c, and its 3' -end region forms a loop by hybridization between B1c and B1. In step (iii) of Fig. 1, FIP is hybridized with the dumbbell-shaped single-stranded DNA product generated in step (ii) of Fig. 1 to be extended, and a new dumbbell-shaped single-stranded DNA product is generated. In step (iv) of Fig. 1, BIP is hybridized and extended using the dumbbell-shaped single-stranded DNA product in step (iii) of Fig. 1 as a template. In steps (iii) and (iv) of Fig. 1, the backward loop primer LB and the forward loop primer LF are each hybridized with a loop region (specifically, a loop between B1 and B2 or a loop between F1 and F2) of the generated dumbbell-shaped DNA product to accelerate the generation of a new DNA product (i.e., amplification).

The LB or LF primer, which is an accelerating primer, binds to a single-stranded region of the above-described dumbbell structure, and specifically, binds to a loop between B1 and B2 or a loop between F1 and F2. The use of LB or LF primers leads to an increase in the origin at which DNA synthesis begins, resulting in a faster reaction rate. In the LAMP reaction, six loops are usually formed in the process, and only two loops are used as the origin of synthesis, but in the case of using LB and LF primers, all six loops are used as the origin of synthesis, so that the amount of DNA synthesized for a predetermined time increases.

A more detailed description of the features and functions of each F3 primer, B3 primer, FIP primer, BIP primer, loop F primer (LF), and loop B (LB) primer may be referred to Notomi et al. (T. Notomi, H. Okayama, H. Masubuchi, T. Yonekawa, K. Watanabe, N. Amino and T. Hase, Nucleic Acids Res., 2000, 28, E63), and Nagamine et al. (K. Nagamine, T. Hase and T. Notomi, Mol. Cell. Probes, 2002, 16, 223-229).

Suitable lengths of primers used in LAMP, including the tagged loop primer of the present disclosure, can be determined in consideration of a number of factors, such as temperature, application, primer source and length of final amplification product, etc. For example, the length of the primer may be at least 8 nucleotides, 9 nucleotides, 10 nucleotides, 15 nucleotides, 20 nucleotides, 25 nucleotides, or 30 nucleotides. Specifically, F3 and B3 may be at least 8 nucleotides, 9 nucleotides, 10 nucleotides, 11 nucleotides, 12 nucleotides, 13 nucleotides, 14 nucleotides, or 15 nucleotides in length, FIP and BIP may be at least 25 nucleotides, 26 nucleotides, 27 nucleotides, 28 nucleotides, 29 nucleotides, or 30 nucleotides in length, and conventional untagged LF and LB may be at least 11 nucleotides, 12 nucleotides, 13 nucleotides, 14 nucleotides, or 15 nucleotides in length, but are not limited thereto.

According to an embodiment, the primer set may be designed using various design software such as commercially available primer design software, for example, Primer Explorer V5 (Fujitsu, Japan), LAVA (LAMP Assay Versatile Analysis), and LAMP Designer (PRIMER Biosoft), in consideration of the characteristics of the LAMP reaction and the characteristics of the primer.

As used herein, the term "nucleic acid" , "nucleotide sequence" or "nucleic acid molecule" refers to a single- or double-stranded deoxyribonucleotide or ribonucleotide polymer in which the nucleotides include derivatives of naturally occurring nucleotides, non-naturally occurring nucleotides, or modified nucleotides, which are capable of functioning in the same manner as naturally occurring nucleotides.

The term "target nucleic acid", "target nucleic acid sequence", or "target sequence", as used herein, refers to a nucleic acid sequence to be detected. The target nucleic acid sequence may be hybridized with the tagged loop primer for the loop-mediated isothermal amplification reaction of the present disclosure. More specifically, in the target nucleic acid of the present disclosure, deoxyribonucleotides (DNAs), ribonucleotides (RNAs), and mixtures or combinations thereof may be used as types of nucleic acids. Bases constituting the nucleic acid may be naturally occurring nucleotides, for example, guanine (G), adenine (A), thymine (T), cytosine (C), and uracil (U) or may include other natural and artificial modified bases. Here, the "modified base" means a base in which the five nucleotides have undergone chemical modification. Examples of the modified base include, but not limited to, methylcytidine, pseudouridine, 4-thiouridine, dihydrouridine, queuosine, and hypoxanthine (inosine (I)). In the present disclosure, the target nucleic acid needs to be a single-stranded chain when detected, but a nucleic acid of a double-stranded or higher-order structure may be used after conversion into a single-stranded chain by heat denaturation, alkali denaturation, or the like. The target nucleic acid of the present disclosure also includes such denatured forms. In addition, the target nucleic acid of the double-stranded structure may be dissociated into the single-stranded structure by an enzymatic reaction using an enzyme such as helicase or an enzyme having strand-displacement activity with respect to the double-stranded chain. For example, when a specialized DNA polymerase enzyme having high strand displacement activity, e.g., *Bacillus stearothermophilus* (Bst) DNA polymerase, *Thermus thermophilus* (Tth) DNA polymerase, *Thermus aquaticus* (Taq) DNA polymerase, *Thermococcus litoralis* DNA polymerase; and *Bacillus caldotenax* DNA polymerase, is used that meets two major requirements: (i) possessing strand displacement activity and (ii) being capable of initiating polymerization reactions at a temperature (e.g., 50°C to 75°C) where thermodynamic activity is maintained so that the reaction product, the polymer, can form a ring structure, the extension reaction can proceed while dissociating the double-stranded DNA in the direction of progress, the target nucleic acid is not limited to a single-stranded sequence. In addition, cDNA prepared by reverse transcription reaction using RNA as a template is also included in the "target nucleic acid" .

In an embodiment, the target nucleic acid may be a nucleic acid of a human, animal, plant, or microorganism. The microorganism may be fungi, protozoa, bacteria, viruses, or algae.

In an embodiment, the target nucleic acid may be a viral nucleic acid, and specifically, the target nucleic acid may be an RNA viral nucleic acid.

In an embodiment, the target nucleic acid may be a respiratory virus nucleic acid. For example, the target nucleic acid may be an influenza virus nucleic acid, a respiratory syncytial virus (RSV) nucleic acid, an adenovirus nucleic acid, an enterovirus nucleic acid, a parainfluenza virus nucleic acid, a metapneumovirus (MPV) nucleic acid, a bocavirus nucleic acid, a rhinovirus nucleic acid, and/or a coronavirus nucleic acid, but is not limited thereto. More specifically, the target nucleic acid may be a nucleic acid of SARS-CoV-2.

In an embodiment, the nucleic acid of SARS-CoV-2 may be selected from the group consisting of E gene, N gene, RdRP gene, S gene, and a combination thereof.

According to an embodiment, when the target nucleic acid is RNA, the isothermal amplification reaction may include a reverse transcription reaction step. The details thereof are disclosed in Joseph Sambrook, et al., Molecular Cloning, A Laboratory Manual, Cold Spring Harbor Laboratory Press, Cold Spring Harbor, N.Y. (2001); and Noonan, K.F. et al., Nucleic Acids Res. 16:10366 (1988).

A reverse transcription reaction is a reaction of synthesizing cDNA from RNA by a reverse transcription reaction using reverse transcriptase. The reverse transcription reaction may be performed in the same tube as the LAMP reaction (one step reaction), and cDNA may be generated first through a separate reverse transcription reaction and then used in the LAMP reaction step (two step reaction). The one-step reaction may be effectively used for high throughput or rapid detection. In addition, when a more precise analysis is required, a two-step reaction may be preferably used.

As used herein, the term "reverse transcriptase" is also referred to as RNA-dependent DNA polymerase, and refers to an enzyme that uses RNA as a template and synthesizes complementary DNA thereto.

In an embodiment, the reverse transcriptase may be originated from various sources, for example, Avian Myeloblastosis Virus-derived Reverse Transcriptase (AMV RTase), Murine Leukemia Virus-derived Reverse Transcriptase (MuLV RTase), and Rous-Associated Virus 2 Reverse Transcriptase (RAV-2 RTase), but is not limited thereto.

Reverse transcriptase requires a primer for synthesizing cDNA from an RNA template. There are largely three types of primers for use in reverse transcriptase reaction: (i) an oligo dT primer that is annealed to poly A tail in mRNA to allow for the synthesis of cDNA from the 3' terminus; (ii) a random primer that is made of random nucleotides 6-9 nt long and allows cDNA synthesis to start at any site of RNA; and (iii) a target-specific primer for synthesizing only a target cDNA.

In an embodiment, the 5-tagging portion of the tagged loop primer of the present disclosure forms a spatially folded structure when the target nucleic acid is absent, so that the reporter molecule and the quencher molecule are in close proximity to each other (see Fig. 2 (b)). When the reporter molecule and the quencher molecule are in close proximity to each other, the quenching effect occurs and the signaling effect by the reporter molecule does not occur. An assimilating probe used for real-time monitoring of a target nucleic acid in a conventional LAMP reaction include two oligonucleotide strands, each comprising a reporter molecule or a quencher molecule, and is based on the principle that a quenching effect occurs when two oligonucleotides form a complementary bond. The present disclosure is characterized in that it uses a single-stranded loop primer comprising an interactive dual labeling system, other than a conventional assimilating probe. In the case of a conventional assimilating probe, a process of hybridizing two probes into a pair of probes by gradually decreasing the temperature from high temperature to low temperature before a LAMP reaction for nucleic acid amplification is required. In addition, in the case of a design of probes consisting of a pair of two oligonucleotides, there is a problem that there are many factors to consider, such as the number of total bonds between nucleotides in consideration of the temperature at which the bonds of two strands of oligonucleotides are denatured into a single-strand, the type and ratio of adenine-guanine and thymine-cytosine bonds, and the like, so that the two oligonucleotides can be maintained in a hybridized state even at the isothermal amplification reaction temperature. In addition, in the case of a conventional assimilating probe, even though it is designed in consideration of the above-described numerous factors, there is a possibility that the complementary bond of two oligonucleotide strands comprising a reporter molecule or a quencher molecule may be partially disrupted, and thus, a signal that is not specific to a target nucleic acid may be generated. Furthermore, there is an inconvenience of having to design two oligonucleotides for probe preparation. On the other hand, the tagged loop primer having the single-stranded interactive dual labeling system according to the present disclosure may solve the above-described drawbacks of the conventional assimilating probe.

In an embodiment, the 5-tagging portion of the tagged loop primer of the present disclosure forms a spatially unfolded structure when the target nucleic acid is present, so that the reporter molecule and the quencher molecule are apart from each other for unquenching (see (iii) of Fig. 3 (a)). This is the most distinguishing feature of the tagged loop primer of the present disclosure compared to the conventional assimilating probe, and the tagged loop primer of the present disclosure can minimize the occurrence of non-specific signaling to the target nucleic acid.

In an embodiment, the reporter molecule and quencher molecule used in the present disclosure constitute an interactive dual labeling system in which energy is non-radioactively transferred between them.

As a representative interactive label system, a FRET (fluorescence resonance energy transfer) label system includes a fluorescent reporter molecule (donor molecule) and a quencher molecule (acceptor molecule). In FRET, the energy donor is fluorescent, but the energy acceptor may be fluorescent or non-fluorescent. In another form of interactive label systems, the energy donor is non-fluorescent, e.g., a chromophore, and the energy acceptor is fluorescent. In yet another form of interactive label systems, the energy donor is luminescent, e.g., bioluminescent, chemiluminescent, or electrochemiluminescent, and the acceptor is fluorescent.

Interactive dual labels include the label pair providing detectable signal based on contact-mediated quenching (Salvatore et al., Nucleic Acids Research, 2002 (30) no. 21 e122 and Johansson et al., J. AM. CHEM. SOC 2002 (124) pp 6950-6956). The interactive label system includes any or all cases in which signals are changed by interaction between at least two molecules (e.g., dyes).

The reporter and the quencher molecules useful for single or dual labels may include any molecules known in the art. Their examples include: Cy2^{™} (506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Phycoerythrin R&B (575), Rhodamine Phalloidin (575), (576), Pyronin Y (580), Rhodamine B (580), TAMRA (582), Rhodamine Red^{™} (590), Cy3.5^{™} (596), ROX (608), Alexa^{™} 594 (615), Texas Red (615), Nile Red (628), YO-PRO^{™}-3 (631), YOYO^{™}-3 (631), R-phycocyanin (642), C-Phycocyanin (648), TO-PRO^{™}-3 (660), TOTO3 (660), DiD DilC(5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705), and Quasar 705 (610). The numbers in parentheses represent maximum emission wavelengths in nanometer.

Suitable pairs of reporter-quencher are disclosed in many literatures as follows: Pesce et al., editors, Fluorescence Spectroscopy (Marcel Dekker, New York, 1971); White et al., Fluorescence Analysis: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996), and U. S. Patent Nos. 3,996,345 and 4,351,760.

Each of the reporter molecule and the quencher molecule may be fluorescent. In addition, the reporter molecule may be fluorescent, whereas the quencher molecule may be non-fluorescent. For example, a non-fluorescent dark quencher capable of quenching fluorescence of a wide range wavelength or a specific wavelength may be used in the present disclosure. When the quencher molecule is fluorescent, the target nucleic acid sequence may be detected from a signal change of the fluorescent quencher molecule.

Reporter molecules and quencher molecules can be linked to oligonucleotides according to conventional methods. For example, the reporter molecule and quencher molecule can be linked to the probe via a spacer comprising at least 3 carbon atoms (e.g., 3-carbon spacer, 6-carbon spacer, 9-carbon spacer, or 12-carbon spacer).

As used herein, the expression "formation of a spatially folded structure" means that the 5'-tagging portion to which the reporter molecule and the quencher molecule are linked has a conformationally, randomly folded shape, and specifically for example, a folded structure such as a random coil, but does not mean that the 5'-tagging portion is linear with a certain direction, or is arranged in a three-dimensional space in a helical shape characteristic of double-stranded nucleic acids.

In an embodiment, when the target nucleic acid is absent, the 5-tagging portion of the tagged loop primer of the present disclosure may form a hairpin structure so that the reporter molecule and the quencher molecule are in close proximity to each other (see Fig. 2 (c)).

In an embodiment, when the target nucleic acid is present, the hairpin structure of the 5-tagging portion of the tagged loop primer of the present disclosure is disrupted, and the reporter molecule and quencher molecule are apart from each other for unquenching (see (iii) of Fig. 3 (b)).

The term "hairpin structure" as used herein has the same meaning as a stem-loop structure, and refers to a structure in which complementary bonds are formed in a single-strand nucleic acid. When the hairpin structure is formed, the distance between nucleic acids apart from each other at a long distance is narrowed.

As described above, the tagged loop primer according to the present disclosure comprises both the reporter molecule and the quencher molecule in the 5-tagging portion, and when the target nucleic acid is absent, the reporter molecule and the quencher molecule are in close proximity to each other through the formation of a spatially folded structure or a hairpin structure, resulting in a quenching effect. More preferably, the 5-tagging portion has a significant advantage in that quenching occurs by forming a spatially folded structure, such as a random coil, rather than forming a hairpin structure. In the case of a conventional assimilating probe, as mentioned above, the binding of two nucleic acid strands forming a double strand is dependent on the isothermal amplification reaction temperature, is not 100% hybridized at the isothermal amplification reaction temperature, and is likely to be partially disrupted to be denatured into a single-strand, which may result in unquenching and then generating a signal, and this implies the possibility of double-strand bond disruption that is not specific to the target nucleic acid, and that a certain amount of bias may occur. According to an embodiment of the present disclosure, in the case of a method of forming a stem-loop structure, even when the bond in the stem is disrupted, since the complementary sequence exists in a single-strand and is fixed at a predetermined distance, the stem structure may be easily reformed and the possibility of bias occurrence may be significantly lowered as compared to an assimilating probe.

In addition, compared to the method of forming the stem-loop structure, a method of forming a spatially folded structure, for example, a random coil, according to another embodiment of the present disclosure is not based on signal generation by a double-stranded bond disruption, thereby avoiding bias that may occur in an assimilating probe or stem-loop structure.

The tagged loop primer of the present disclosure does not require any specific lengths.

In an embodiment, the tagged loop primer of the disclosure may be 15-150 nucleotides, 15-140 nucleotides, 15-130 nucleotides, 15-120 nucleotides, 15-110 nucleotides, 15-100 nucleotides, 15-90 nucleotides, 15-80 nucleotides, 15-70 nucleotides, 15-60 nucleotides, 15-50 nucleotides, 15-40 nucleotides, 20-150 nucleotides, 20-140 nucleotides, 20-130 nucleotides, 20-120 nucleotides, 20-110 nucleotides, 20-100 nucleotides, 20-90 nucleotides, 20-80 nucleotides, 20-70 nucleotides, 20-60 nucleotides, 20-50 nucleotides, 20-40 nucleotides, 30-150 nucleotides, 30-140 nucleotides, 30-130 nucleotides, 30-120 nucleotides, 30-110 nucleotides, 30-100 nucleotides, 30-90 nucleotides, 30-80 nucleotides, 30-70 nucleotides, 30-60 nucleotides, 30-50 nucleotides, 30-40 nucleotides, 35-100 nucleotides, 35-90 nucleotides, 35-80 nucleotides, 35-70 nucleotides, 35-60 nucleotides, or 35-50 nucleotides in length.

In an embodiment, the 3' -targeting portion of the tagged loop primer of the present disclosure has a length sufficient to be specifically hybridized with the target nucleic acid under conditions of loop-mediated isothermal amplification (LAMP) reactions.

In an embodiment, the 3' -targeting portion of the tagged loop primer of the present disclosure is 10-100 nucleotides, 10-90 nucleotides, 10-80 nucleotides, 10-70 nucleotides, 10-60 nucleotides, 10-50 nucleotides, 10-40 nucleotides, 10-30 nucleotides, 10-20 nucleotides, 12-100 nucleotides, 12-90 nucleotides, 12-80 nucleotides, 12-70 nucleotides, 12-60 nucleotides, 12-50 nucleotides, 12-40 nucleotides, 12-30 nucleotides, 12-20 nucleotides, 15-100 nucleotides, 15-90 nucleotides, 15-80 nucleotides, 15-70 nucleotides, 15-60 nucleotides, 15-50 nucleotides, 15-40 nucleotides, 15-30 nucleotides, or 15-20 nucleotides in length.

In an embodiment, the 3' -targeting portion of the tagged loop primer of the present disclosure may have a Tm value of 40°C to 70°C, and specifically, may have a Tm value of 45°C to 70°C, 50°C to 70°C, 55°C to 70°C, 60°C to 70°C, 40°C to 65°C, 45°C to 65°C, 50°C to 65°C, 55°C to 65°C, or 60°C to 65°C, but is not limited thereto.

In an embodiment, the 5' -tagging portion of the tagged loop primer of the present disclosure has a nucleotide length such that the reporter molecule and the quencher molecule therein are apart from each other to allow the quencher molecule to unquench the signal from the reporter molecule.

In an embodiment, the 5' -tagging portion of the tagged loop primer of the present disclosure is at least 5 nucleotides in length, specifically at least 10 nucleotides in length. More specifically, the 5' -tagging portion may be 5-50 nucleotides, 5-45 nucleotides, 5-40 nucleotides, 5-35 nucleotides, 5-30 nucleotides, 5-25 nucleotides, 5-24 nucleotides, 5-23 nucleotides, 5-22 nucleotides, 5-21 nucleotides, 5-20 nucleotides, 8-50 nucleotides, 8-45 nucleotides, 8-40 nucleotides, 8-35 nucleotides, 8-30 nucleotides, 8-25 nucleotides, 8-24 nucleotides, 8-23 nucleotides, 8-22 nucleotides, 8-21 nucleotides, 8-20 nucleotides, 10-50 nucleotides, 10-45 nucleotides, 10-40 nucleotides, 10-35 nucleotides, 10-30 nucleotides, 10-25 nucleotides, 10-24 nucleotides, 10-23 nucleotides, 10-22 nucleotides, 10-21 nucleotides, 10-20 nucleotides, 12-50 nucleotides, 12-45 nucleotides, 12-40 nucleotides, 12-35 nucleotides, 12-30 nucleotides, 12-25 nucleotides, 12-24 nucleotides, 12-23 nucleotides, 12-22 nucleotides, 12-21 nucleotides, 12-20 nucleotides, 14-50 nucleotides, 14-45 nucleotides, 14-40 nucleotides, 14-35 nucleotides, 14-30 nucleotides, 14-25 nucleotides, 14-24 nucleotides, 14-23 nucleotides, 14-22 nucleotides, 14-21 nucleotides, 14-20 nucleotides, 15-50 nucleotides, 15-45 nucleotides, 15-40 nucleotides, 15-35 nucleotides, 15-30 nucleotides, 15-25 nucleotides, 15-24 nucleotides, 15-23 nucleotides, 15-22 nucleotides, 15-21 nucleotides, 15-20 nucleotides, 16-50 nucleotides, 16-45 nucleotides, 16-40 nucleotides, 16-35 nucleotides, 16-30 nucleotides, 16-25 nucleotides, 16-24 nucleotides, 16-23 nucleotides, 16-22 nucleotides, 16-21 nucleotides, or 16-20 nucleotides in length.

In one embodiment, one of the reporter molecule and the quencher molecule is located at the 5' -end or 1-5 nucleotides apart from the 5' -end of the 5' -tagging portion of the present invention, and the other is located to quench or unquench the signal from the reporter molecule depending on the conformation of the fragment. More specifically, when the 5' -tagging portion has a folded conformation under a general condition, the distance between the reporter molecule and the quencher molecule becomes shorter and a quenching effect occurs, but when the 5' -tagging portion is unfolded by double strand formation in the 5' -tagging portion, the reporter and quencher molecules are sufficiently spaced apart for unquenching.

In an embodiment, one of the reporter molecule and the quencher molecule is linked to a 5' -end of the 5' -tagging portion, and the other is linked to an internal deoxythymidine (dT) present in the 5' -tagging portion. As described above, the internal dT refers to an internal dT spaced apart from the 5' -end of the 5' -tagging portion sufficient to quench or unquench a signal from a reporter molecule depending on the conformation of the fragment.

Any of the reporter molecule and quencher molecule may be linked to the 5' -end or to a position 1-5 nucleotides apart from the 5' -end of the 5' -tagging portion and such linking does not significantly affect the effect of the invention.

As used herein, the term "end" refers to the last nucleotide, e.g., the 5' -end refers to the last nucleotide in the 5' direction in the sequence, and the 3' -end refers to the last nucleotide in the 3' direction in the sequence.

In addition, the term "end portion" as used herein refers to a portion comprising the last nucleotide in a sequence and several or dozens of nucleotides adjacent thereto.

In an embodiment, the reporter molecule and the quencher molecule are located at most 50 nucleotides apart from each other, and more specifically, for example, at most 45 nucleotides, at most 40 nucleotides, at most 35 nucleotides, at most 30 nucleotides, or at most 25 nucleotides apart from each other. According to a preferred embodiment, the reporter molecule and the quencher molecule are located at least 4 nucleotides apart, specifically at least 5 nucleotides, at least 6 nucleotides, at least 7 nucleotides, at least 8 nucleotides, at least 9 nucleotides, more specifically at least 10 nucleotides, at least 11 nucleotides, at least 12 nucleotides, at least 13 nucleotides, at least 14 nucleotides, or at least 15 nucleotides apart.

In an embodiment, the reporter molecule and the quencher molecule are located 5-45 nucleotides, 5-40 nucleotides, 5-35 nucleotides, 5-30 nucleotides, 5-25 nucleotides, 5-24 nucleotides, 5-23 nucleotides, 5-22 nucleotides, 5-21 nucleotides, 5-20 nucleotides, 8-45 nucleotides, 8-40 nucleotides, 8-35 nucleotides, 8-30 nucleotides, 8-25 nucleotides, 8-24 nucleotides, 8-23 nucleotides, 8-22 nucleotides, 8-21 nucleotides, 8-20 nucleotides, 10-45 nucleotides, 10-40 nucleotides, 10-35 nucleotides, 10-30 nucleotides, 10-25 nucleotides, 10-24 nucleotides, 10-23 nucleotides, 10-22 nucleotides, 10-21 nucleotides, 10-20 nucleotides, 12-45 nucleotides, 12-40 nucleotides, 12-35 nucleotides, 12-30 nucleotides, 12-25 nucleotides, 12-24 nucleotides, 12-23 nucleotides, 12-22 nucleotides, 12-21 nucleotides, 12-20 nucleotides, 14-45 nucleotides, 14-40 nucleotides, 14-35 nucleotides, 14-30 nucleotides, 14-25 nucleotides, 14-24 nucleotides, 14-23 nucleotides, 14-22 nucleotides, 14-21 nucleotides, 14-20 nucleotides, 15-45 nucleotides, 15-40 nucleotides, 15-35 nucleotides, 15-30 nucleotides, 15-25 nucleotides, 15-24 nucleotides, 15-23 nucleotides, 15-22 nucleotides, 15-21 nucleotides, or 15-20 nucleotides apart from each other.

According to another aspect, the present disclosure provides a method of detecting a target nucleic acid, comprising performing a loop-mediated isothermal amplification (LAMP) reaction using one or more of the above-described tagged loop primers.

In the case of the tagged loop primer, as shown in Fig. 3, either or both of a forward tagged loop primer (tagged LF) and a backward tagged loop primer (tagged LB) may be used, preferably both tagged LF and tagged LB may be used, and in the case of two or more target nucleic acids, one or more of tagged LF and tagged LB suitable for each target nucleic acid may be additionally used to simultaneously detect the two or more target nucleic acids.

In an embodiment, the method of the present disclosure is directed to detection of one target nucleic acid or simultaneous detection of two or more target nucleic acids. When the method of the present disclosure is applied for simultaneous detection of two or more target nucleic acids, all primer sets suitable for each target should be included. The primer set in an embodiment refers to four primers (F3, B3, FIP, BIP) essential for the LAMP reaction and one or more tagged loop primers (one or more of tagged LF and tagged LB) of the present disclosure, and the above-described primer set should be prepared for each target nucleic acid. In addition, at least one of two untagged loop primers (LF and LB), which have been generally used in the art, may be additionally included in order to improve the reaction rate.

In an embodiment, the isothermal temperature condition of the method of the present disclosure is any one temperature selected from 50°C to 75°C, specifically, any one temperature selected from 50°C to 70°C, any one temperature selected from 50°C to 65°C, any one temperature selected from 55°C to 75°C, any one temperature selected from 55°C to 70°C, any one temperature selected from 55°C to 65°C, any one temperature selected from 60°C to 75°C, any one temperature selected from 60°C to 70°C, or any one temperature selected from 60°C to 65°C. In certain embodiments, the isothermal temperature condition is 60°C, 61°C, or 62°C, and more particularly 62°C, but not limited thereto.

In an embodiment, the method of the present disclosure is performed on DNA, cDNA or RNA nucleic acids.

In an embodiment, the target nucleic acid may be a nucleic acid of a human, animal, plant, or microorganism. The microorganism may be fungi, protozoa, bacteria, viruses, or algae.

In an embodiment, the target nucleic acid may be a viral nucleic acid, and specifically, the target nucleic acid may be an RNA viral nucleic acid.

In a specific embodiment, the target nucleic acid may be a respiratory virus nucleic acid. For example, the target nucleic acid may be an influenza virus nucleic acid, a respiratory syncytial virus (RSV) nucleic acid, an adenovirus nucleic acid, an enterovirus nucleic acid, a parainfluenza virus nucleic acid, a metapneumovirus (MPV) nucleic acid, a bocavirus nucleic acid, a rhinovirus nucleic acid, and/or a coronavirus nucleic acid, but is not limited thereto. More specifically, the target nucleic acid may be a nucleic acid of SARS-CoV-2.

In an embodiment, the nucleic acid of SARS-CoV-2 may be selected from the group consisting of E gene, N gene, RdRP gene, S gene, and a combination thereof.

According to an embodiment, when the target nucleic acid is RNA, the isothermal amplification reaction may comprise a reverse transcription reaction step. The details thereof are described above.

When the method for detecting a target nucleic acid according to the present disclosure is used, isothermal amplification is monitored in real time using a nucleic acid detection device that can detect fluorescent dyes, etc. in real time during the RT-LAMP reaction without additional electrophoresis or use of SYBR Green I after the reaction, and amplification of the target nucleic acid can be confirmed through annealing peak.

In an embodiment, the step of performing the loop-mediated isothermal amplification (LAMP) reaction of the present disclosure may be performed using a tagged loop primer and an untagged loop primer together. As used herein, the term "untagged loop primer" refers to loop primers LB and LF that have been conventionally used to improve the reaction rate of the LAMP reaction. It is preferred that the untagged loop primer does not include a 5' -tagged portion, unlike the tagged loop primer of the present disclosure. Using an untagged loop primer together with the tagged loop primer of the present disclosure helps increase the sensitivity of target nucleic acid detection by further improving the reaction rate in the LAMP reaction. It is understood that the untagged loop primer can be easily designed by those skilled in the art by a known design method, and is not particularly limited.

The method for detecting a target nucleic acid of the present disclosure has the technical feature of using the tagged loop primer, which is another aspect of the present disclosure described above, and redundant contents are omitted to avoid excessive complexity of the description of the present specification.

According to another aspect, the present disclosure provides a composition for a loop-mediated isothermal amplification (LAMP) reaction comprising the tagged loop primer described above.

The composition for a loop-mediated isothermal amplification reaction of the present disclosure may be used to perform a loop-mediated isothermal amplification reaction by mixing with a lysate of a detection sample.

According to an embodiment, the composition for a loop-mediated isothermal amplification (LAMP) reaction may comprise a composition for a reverse transcription reaction.

According to an embodiment, the composition for reverse transcription reaction may comprise a reverse transcription polymerase and/or a reverse transcription primer.

According to an embodiment, the reverse transcription primer may be an oligo dT-primer, a random primer, or a target specific primer.

According to an embodiment, the reverse transcription primer may be a target specific primer.

According to an embodiment, the target-specific primer may be used to synthesize cDNA in a reverse transcription reaction, and then used as a primer pair for amplification of the obtained cDNA by constructing a primer pair or a primer set with another primer(s) in a target nucleic acid sequence amplification reaction.

According to an embodiment, the composition for LAMP reaction may further comprise reverse transcriptase, DNA polymerase, dNTPs, buffer and/or magnesium, and the like.

According to an embodiment, the DNA polymerase is a polymerase derived from thermophilic microorganisms, and may particularly include a polymerase lacking a 5' ->3' exonuclease function. For example, the polymerase may include *Bacillus stearothermophilus* (Bst) DNA polymerase, *Thermus thermophilus* (Tth) DNA polymerase, *Thermus aquaticus* (Taq) DNA polymerase, *Thermococcus litoralis* DNA polymerase; and *Bacillus caldotenax* DNA polymerase, but is not limited thereto.

In an embodiment, the DNA polymerase may be a Bst DNA polymerase. However, it is noted that other DNA polymerases may be used, as long as they meet two major requirements: (i) possessing strand displacement activity and (ii) being capable of initiating polymerization reactions at a temperature (e.g., 50°C to 75°C) where thermodynamic activity is maintained so that the resulting polymer can form a ring structure. In addition, for the RT-LAMP reaction, GspSSD DNA polymerase, which has faster amplification ability and stronger reverse transcriptase activity than Bst DNA polymerase mainly used in the existing LAMP reaction, may be used, but is not limited thereto.

According to an embodiment, the composition for LAMP reaction may further comprise a nucleotide analog instead of dNTPs. Nucleotide analogs are those modified or not naturally occurring, which can be polymerized alone or in combination with a natural nucleotide in a template-driven DNA synthesis process.

According to an embodiment, examples of the buffer include, but not limited to, a sodium phosphate buffer, a potassium phosphate buffer, a Tris-HCl buffer, or a Tricine buffer.

According to an embodiment, the magnesium may be used in the form of a salt such as magnesium acetate, magnesium chloride, or magnesium sulfate.

In the present disclosure, the composition for a loop-mediated isothermal amplification reaction may optionally include reagents required for performing target amplification reactions (e.g., LAMP reaction) such as nucleic acid polymerase, buffers, polymerase cofactors, and deoxyribonucleotide-5-triphosphates. Optionally, the composition for LAMP reaction may further include various polynucleotide molecules, reverse transcriptase, various buffers and reagents, and antibodies that inhibit nucleic acid polymerase activity. The composition for LAMP reaction may further include oligonucleotides or reagents necessary for performing positive control reactions. Optimal amounts of reagents to be used in a given reaction can be readily determined by one of ordinary skill in the art having the benefit of the present disclosure. The components as describe above may be present in separate containers or a single container.

According to another aspect, the present disclosure provides a kit for detecting a target nucleic acid, comprising the above-described loop-mediated isothermal amplification (LAMP) composition.

The kit for detecting a target nucleic acid of the present disclosure may include a container for storing/transporting the composition, tools and instruments required for detecting a target nucleic acid, in addition to the composition for detecting a target nucleic acid.

Hereinafter, the present invention will be described in more detail with reference to Examples. These Embodiments are intended to describe the present invention in more detail, and it will be apparent to those skilled in the art that the scope of the present invention set forth in the appended claims is not limited by these Examples.

### Mode for Invention

### EXAMPLES

### Example: Detection of target nucleic acid sequences

It was examined whether the tagged loop primer according to the present disclosure can be used to detect a single or a plurality of target nucleic acids.

### 1. Preparation of target nucleic acids

The RdRP gene and/or N gene of SARS-CoV-2 was used as a target nucleic acid, and for this, a SARS-CoV-2 synthetic genome (Twist Synthetic SARS-CoV-2 RNA control 2, Twist Bioscience, Cat. No. 102024) composed of RNA was used as a template. The SARS-CoV-2 synthetic genome prepared at a concentration of 10⁶ copies/µL was serially diluted 10 times with distilled water, thereby preparing three concentrations (concentration 1: 4×10³ copies/µL, concentration 2: 4×10² copies/µL, concentration 3: 4×10¹ copies/µL) of the SARS-CoV-2 synthetic genome.

RNase P was used as an internal control, and for this, Total RNA Control (Human) (Applied Biosystems, Cat. No. 4307281) extracted from cells was used as an internal control template. The total RNA prepared at a concentration of 50 ng/µL was serially diluted 10 times with distilled water, thereby preparing two concentrations (concentration 1: 4×10⁻² ng/µL, concentration 2: 4×10⁻³ ng/µL) of the total RNA.

Distilled water was used as a negative control.

### 2. Preparation of oligonucleotides

Primer sets (F3, B3, FIP, BIP, LF, and LB) and loop primers according to the present disclosure for detecting each target nucleic acid were prepared as shown in Table 1 below.

**[Table 1]**

| Sequences of oligonucleotides | | | |
|---|---|---|---|
| SEQ ID No. | Gene | Oligo type | Sequence (5' to 3') |
| 1 | RdRp | F3 | ACTGACTTAACAAAGCCTTAC |
| 2 | RdRp | B3 | CACCATCAACAAATATTTTTCTCAC |
| 3 | RdRp | FIP | TGGGTGGTATGTCTGATCCCAATA- GGGATTTGTTAAAATATGACTTCAC |
| 4 | RdRp | BIP | TGTGTTAACTGTTTGGATGACAGA-GGTCCAAAACTTGTAGGTGG |
| 5 | RdRp | LF | ACGGTCAAAGAGTTTTAACCTCTC |
| 6 | RdRp | LB | TGCATTCTGCATTGTGCAAAC |
| 7 | RdRp | Tagged loop primer | CalRed610/CTCGCGAACGTATGCTGTGC/T(BHQ- 2)/CCTCGTGGATAGGCACGGTCAAAGAGTTTTAACCTCTC |
| 8 | N | F3 | CAAGCTTTCGGCAGACGT |
| 9 | N | B3 | GGATCTTTGTCATCCAATTTGATGG |
| 10 | N | FIP | TGCGGCCAATGTTTGTAATCAGTTCTCCAGAACAAACCCAAGGAAA T |
| 11 | N | BIP | CCAGCGCTTCAGCGTTCTTCGACCTGTGTAGGTCAACCAC |
| 12 | N | LF | TGTCTGATTAGTTCCTGGTCCCCAA |
| 13 | N | LB | TCGCGCATTGGCATGGAAGT |
| 14 | N | Tagged loop primer | Quasar670/CGACGGATGCAACGTAGCGA/T(BHQ- 2)/CATGCAACGTACGCTCGCGCATTGGCATGGAAGT |
| 15 | RNase P | F3 | TTCAAGGGCCCGGCTCTA |
| 16 | RNase P | B3 | GGCACTGGAAATTGTATACCTTCTC |
| 17 | RNase P | FIP | ACAGTTATGCAGACTAAATCCACATTGTTGTTGCAGTTTTTCCAAA GACA |
| 18 | RNase P | BIP | ACCATTTTACTTCAAAAGACCTCCTAGCAGGGCTATAGACAAGTTC A |
| 19 | RNase P | LF | TGTGTGCAAGCAATATGAAAAAGCT |
| 20 | RNase P | LB | TTGACCGAGGCCTGGCTT |
| 21 | RNase P | Tagged loop primer | CalOrange560AGTCGACCGTACTGTCGTAC/T(BHQ- 2)/CGTCGCGCGAACGTTTGACCGAGGCCTGGCTT |

### 3. Detection of a target nucleic acid

### (1) Mono RT-LAMP

As Mono RT-LAMP reagents for each the RdRp gene, the N gene, and the RNase P, the final 15 µL of Mono RT-LAMP reagents were prepared respectively, which included 4 pmole of F3 (SEQ ID NO: 1, SEQ ID NO: 8, or SEQ ID NO: 15), 4 pmole of B3 (SEQ ID NO: 2, SEQ ID NO: 9, or SEQ ID NO: 16), 32 pmole of FIP (SEQ ID NO: 3, SEQ ID NO: 10, or SEQ ID NO: 17), 32 pmole of BIP (SEQ ID NO: 4, SEQ ID NO: 11, or SEQ ID NO: 18), 8 pmole of LF (SEQ ID NO: 5, SEQ ID NO: 12, or SEQ ID NO: 19), 8 pmole of LB (SEQ ID NO: 6, SEQ ID NO: 13, or SEQ ID NO: 20), 8 pmole of a tagged loop primer (SEQ ID NO: 7, SEQ ID NO: 14, SEQ ID NO: 21), 0.4 µL of WarmStart RTx Reverse Transcriptase (NEB, Cat. No. M0380L), 0.8 µL of *Bst* 2.0 WarmStart DNA polymerase (NEB, Cat. No. M0538L), 2.8 µL of 10 mM of dNTP Mix, 1.2 µL of 100 mM of MgSO₄, and 1 µL of 10X Isothermal Amplification Buffer (NEB, B0537S).

2.5 µL of each of the three concentrations (two concentrations in the case of RNase P) of target nucleic acid prepared in Example 1 and 2.5 µL of distilled water were mixed with 15 µL of each of the Mono RT-LAMP reagents prepared for each target nucleic acid to prepare a reaction mixture having a final volume of 20 µL. The tubes containing each reaction mixture was placed in a real-time thermal cycler (CFX96, Bio-Rad) and reacted at 62°C for 20 minutes, and the signal was measured every 30 seconds. Ct values were calculated by Auto calculated single threshold, and all experiments were performed in duplicate.

A negative control reaction was also performed under the same conditions as described above by using a reaction mixture in which 5 µL of distilled water was added instead of the target nucleic acid.

As a result, as shown in Fig. 4, it was found that the RdRp gene as a target nucleic acid was detected with an average Ct value of 15.5 for concentration 1, an average Ct value of 18.2 for concentration 2, and an average Ct value of 25.8 for concentration 3, but the negative control was not detected in any of experiments in duplicate.

As shown in Fig. 5, it was found that the N gene as another target nucleic acid was detected with an average Ct value of 16.2 for concentration 1, an average Ct value of 23.8 for concentration 2, and a Ct value of 38.18 in only one of experiments in duplicate for concentration 3, but the negative control was not detected in any of experiments in duplicate.

As shown in Fig. 6, it was found that the RNase P gene as an internal control was detected with an average Ct value of 18.4 for concentration 1, a Ct value of 22.83 in only one of experiments in duplicate, but the negative control was not detected in any of experiments in duplicate.

As shown in Figs. 4 to 6, these results indicate that the target nucleic acids can be detected using the tagged loop primer according to the present disclosure.

### (2) Multi RT-LAMP

As Multi RT-LAMP reagent for RdRP gene, N gene, RNase P, the final 15 µL Multi RT-LAMP reagent was prepared, which includes the following components for each target nucleic acid: 4 pmole of F3 (SEQ ID NO: 1, SEQ ID NO: 8 and SEQ ID NO: 15), 4 pmole of B3 (SEQ ID NO: 2, SEQ ID NO: 9 and SEQ ID NO: 16), 32 pmole of FIP (SEQ ID NO: 3, SEQ ID NO: 10 and SEQ ID NO: 17), 32 pmole of BIP (SEQ ID NO: 4, SEQ ID NO: 11 and SEQ ID NO: 18), 8 pmole of LF (SEQ ID NO: 5, SEQ ID NO: 12 and SEQ ID NO: 19), 8 pmole of LB (SEQ ID NO: 6, SEQ ID NO: 13 and SEQ ID NO: 20), 8 pmole of a tagged loop primer (SEQ ID NO: 7, SEQ ID NO: 14 and SEQ ID NO: 21), 0.4 µL of WarmStart RTx Reverse Transcriptase (NEB, Cat. No. M0380L), 0.8 µL of *Bst* 2.0 WarmStart DNA polymerase (NEB, Cat. No. M0538L), 2.8 µL of 10 mM of dNTP Mix, 1.2 µL of 100 mM of MgSO₄, and 1 µL of 10X Isothermal Amplification Buffer (NEB, B0537S).

15 µL of the prepared Multi RT-LAMP reagent was mixed with 2.5 µL (10³ cp/rxn) of the SARS-CoV-2 nucleic acid genome with concentration 2 and 2.5 µL (1 ng/rxn) of the RNase P target nucleic acid with concentration 1, which were prepared in Example 1, to prepare a reaction mixture having a final volume of 20 µL. The tube containing the reaction mixture was placed in a real-time thermal cycler (CFX96, Bio-Rad) and reacted at 62°C for 20 minutes, and the signal was measured every 30 seconds. Ct values were calculated by Auto calculated single threshold, and all experiments were performed in duplicate.

A negative control reaction was also performed under the same conditions as described above by using a reaction mixture in which 5 µL of distilled water was added instead of the target nucleic acid.

As a result, as shown in Fig. 7, it was found that in one reaction tube, the RdRp gene as a target nucleic acid, the N gene as another target nucleic acid, and the RNase P gene as an internal control were all detected, but the negative control was not detected.

These results indicate that a plurality of target nucleic acids can be detected using the tagged loop primer according to the present disclosure.

## Claims

1. A tagged loop primer for detecting a target nucleic acid having, in a 3' to 5' direction, (i) F3c, F2c, F1c, B1, B2 and B3 or (ii) B3c, B2c, B1c, F1, F2 and F3, the tagged loop primer comprising:
(i) a 3' -targeting portion comprising a nucleotide sequence complementary to all or part of a sequence between the B1 and the B2 or between the F1 and the F2 of the target nucleic acid; and
(ii) a 5' -tagging portion comprising a nucleotide sequence non-complementary to the target nucleic acid:
wherein the 5' -tagging portion comprises a reporter molecule and a quencher molecule,
wherein in the absence of the target nucleic acid, the reporter molecule and the quencher molecule in the 5' -tagging portion are in close proximity to each other to allow the quencher molecule to quench a signal from the reporter molecule,
wherein in the presence of the target nucleic acid, the reporter molecule and the quencher molecule in the 5' -tagging portion are separated from each other to allow the quencher molecule to unquench the signal from the reporter molecule.

2. The tagged loop primer of claim 1, wherein in the absence of the target nucleic acid, the 5-tagging portion is spatially folded.

3. The tagged loop primer of claim 2, wherein in the presence of the target nucleic acid, the 5-tagging portion is spatially unfolded.

4. The tagged loop primer of claim 1, wherein in the absence of the target nucleic acid, the 5-tagging portion forms a hairpin structure.

5. The tagged loop primer of claim 4, wherein in the presence of the target nucleic acid, the hairpin structure of the 5-tagging portion is disrupted.

6. The tagged loop primer of claim 1, wherein the tagged loop primer is 20 to 150 nucleotides in length.

7. The tagged loop primer of claim 1, wherein the 3' -targeting portion has a nucleotide length sufficient to hybridize with the target nucleic acid in the LAMP reaction.

8. The tagged loop primer of claim 7, wherein the 3' -targeting portion is 10 to 100 nucleotides in length.

9. The tagged loop primer of claim 1, wherein the 3' -targeting portion has a Tm value of 40°C to 70°C.

10. The tagged loop primer of claim 1, wherein the 5' -tagging portion has a nucleotide length such that the reporter molecule and the quencher molecule therein are apart from each other to allow the quencher molecule to unquench the signal from the reporter molecule.

11. The tagged loop primer of claim 10, wherein the 5' -tagging region is at least 10 nucleotides in length.

12. The tagged loop primer of claim 1, wherein the reporter molecule and the quencher molecule are located at least 10 nucleotides apart from each other.

13. The tagged loop primer of claim 1, wherein one of the reporter molecule and the quencher molecule is linked to a 5' -end of the 5' -tagging portion, and the other is linked to an internal deoxythymidine (dT) present in the 5' -tagging portion.

14. A method for detecting one or more target nucleic acids, comprising performing a loop-mediated isothermal amplification (LAMP) reaction using one or more tagged loop primers according to any one of claims 1 to 13.

15. The method of claim 14, wherein the LAMP reaction is performed at a temperature between 50°C and 75°C.

16. The method of claim 14, wherein the target nucleic acid is from a virus.

17. The method of claim 16, wherein the virus is an RNA virus.

18. The method of claim 14, wherein the LAMP reaction further uses a conventional, untagged loop primer.

19. A composition for a loop-mediated isothermal amplification reaction, comprising a tagged loop primer according to any one of claims 1 to 13.
